# EUROPEAN PATENT APPLICATION

(11) **EP 4 316 454 A1**
(43) Date of publication of application: **07.02.2024**
(21) Application number: 22779431.0
(22) Date of filing: 21.01.2022
(51) Int. Cl.: A61K 6/77, A61K 6/60

(54) **ION SUSTAINED-RELEASE COMPOSITE PARTICLE AND METHOD FOR MANUFACTURING ION SUSTAINED-RELEASE COMPOSITE PARTICLE**

(30) Priority: 30.03.2021 JP 2021058605
(71) Applicant: GC Corporation, Sunto-gun, Shizuoka 410-1307 (JP)
(72) Inventor: NAGANO, Yasuyuki, Tokyo 174-8585 (JP); SHIDA, Enzo, Tokyo 174-8585 (JP); TANAKA, Koji, Tokyo 174-8585 (JP); MORI, Daizaburo, Tokyo 174-8585 (JP)
(74) Representative: Müller-Boré & Partner Patentanwälte PartG mbB
(86) International application number: PCT/JP2022/002286
(87) International publication number: WO 2022/209201

(57) **Abstract**

Ion sustained-release composite particles include ion sustained-release glass and a polymer compound, wherein the polymer compound includes a homo polymer or a copolymer of a (meth)acrylate compound having a hydroxyl group.

## Description

### [Technical Field]

The present invention relates to ion sustained-release composite particles.

### [Background Art]

For example, fluoroaluminosilicate glass is known as ion sustained-release glass. When fluoroaluminosilicate glass is blended into a dental composition, fluorine ions can be sustained-released from the dental composition, which is expected to improve dentin quality. Such dental compositions include, for example, glass ionomer cement.

In addition, as other ion sustained-release glass, the applicant has produced dental glass powders containing zinc, silicon, and fluorine and compositions containing the glass powders (for example, Patent Documents 1 to 4).

### [Prior art documents]

### [Patent Documents]

[Patent document 1] Japanese Patent No. 6783852
[Patent document 2] Japanese Patent No. 6744399
[Patent document 3] Japanese Patent No. 6633750
[Patent document 4] Japanese Patent No. 6687731

### [Summary of Invention]

### [Problem to be solved by the invention]

However, ion sustained-release glass such as fluoroaluminosilicate glass and zinc-containing glass are prone to storage problems and curing time problems when blended into dental compositions due to their high reactivity to acids.

An object of the present invention is to provide ion sustained-release composite particles with suppressed acid reactivity and good ion sustained-release properties.

### [Means for Solving Problems]

The ion sustained-release composite particles according to one aspect of the present invention are ion sustained-release composite particles containing ion sustained-release glass and a polymer compound, wherein the polymer compound contains a homo polymer or a copolymer of a (meth)acrylate compound having a hydroxyl group.

### [Effects of the Invention]

According to one aspect of the present invention, ion sustained-release composite particles with suppressed acid reactivity and good ion sustained-release properties can be provided.

### [Mode for Carrying Out the Invention]

Hereafter, the embodiment of the present invention will be described in detail.

### <Ion Sustained-Release Composite Particles>

The ion sustained-release composite particles according to the present embodiment contain ion sustained-release glass and a polymer compound. In the present specification, ion sustained-release refers to the property that the component contained in the glass is dissolved and gradually released in an ionic state. Composite particles are particles in which the particles of the ion sustained-release glass are composited with a polymer.

The ion sustained-release glass contained in the ion sustained-release composite particles is preferably a glass containing at least one of zinc, calcium, lanthanum, and strontium.

The content of zinc in the ion sustained-release glass is not particularly limited, but for example, the content of zinc oxide (ZnO) in terms of oxide is in a range from 3% by mass to 60% by mass, preferably in a range from 5% by mass to 50% by mass, more preferably in a range from 10% by mass to 40% by mass.

When the content of ZnO in the ion sustained-release glass is 3% by mass or more, the sustained-release of zinc ions in the ion sustained-release glass can be enhanced, and the effect of inhibiting dentin demineralization can be improved. Moreover, when the content of ZnO in the ion sustained-release glass is 60% by mass or less, the viscosity of the ion sustained-release glass can be reduced, and the workability can be improved when the ion sustained-release composite particles are used in a dental composition.

The content of calcium contained in the ion sustained-release glass is not particularly limited, but for example, in the calcium in the ion sustained-release glass, the content of calcium oxide (CaO) in terms of oxide is in a range from 1% by mass to 25% by mass, preferably in a range from 3% by mass to 20% by mass, and more preferably in a range from 5% by mass to 15% by mass.

When the content of CaO in the ion sustained-release glass is 1% by mass or more, the sustained-release of calcium ions in the ion sustained-release glass can be enhanced, and the effect of inhibiting dentin demineralization can be improved. In addition, when the content of CaO in the ion sustained-release glass is 25% by mass or less, the viscosity of the ion sustained-release glass is reduced and the workability can be improved when the ion sustained-release composite particles are used in a dental composition.

The content of lanthanum in the ion sustained-release glass is not particularly limited, but for example, the content of lanthanum oxide (La₂O₃) in terms of oxide is in a range from 5% by mass to 60% by mass, preferably in a range from 15% by mass to 50% by mass, and more preferably in a range from 30% by mass to 40% by mass.

When the content of La₂O₃ in the ion sustained-release glass is 5% by mass or more, the acid resistance of the glass is improved. When the content of La₂O₃ in the ion sustained-release glass is 60% by mass or less, the powder of the ion sustained-release glass can be easily produced.

The content of strontium in the ion sustained-release glass is not particularly limited, but for example, the content of strontium oxide (SrO) in terms of oxide is in a range from 5% by mass to 55% by mass, preferably in a range from 10% by mass to 50% by mass, and more preferably in a range from 20% by mass to 45% by mass.

When the content of SrO in the ion sustained-release glass is 5% by mass or more, the sustained-release of strontium ions in the ion sustained-release glass can be enhanced, and the effect of inhibiting dentin demineralization can be improved. Moreover, when the content of SrO in the ion sustained-release glass is 55% by mass or less, the viscosity of the ion sustained-release glass can be reduced, and the workability can be improved when the ion sustained-release composite particles are used for a dental composition.

The ion sustained-release glass may contain aluminum, silicon, and fluorine as other components.

The content of aluminum in the ion sustained-release glass is not particularly limited, but for example, the content of aluminum oxide (Al₂O₃) in terms of oxide is in a range from 0.1% by mass to 30% by mass, preferably in a range from 0.3% by mass to 25% by mass, and more preferably in a range from 0.5% by mass to 20% by mass.

When the content of Al₂O₃ in the ion sustained-release glass is 0.1% by mass or more, the mechanical strength of a cured body is improved. When the content of Al₂O₃ in the ion sustained-release glass is 30% by mass or less, the powder of the ion sustained-release glass can be easily produced.

In addition, the content of silicon (Si) in the ion sustained-release glass is not particularly limited, but for example, in the silicon in the ion sustained-release glass, the content of silicon oxide (SiO₂) in terms of oxide is preferably in a range from 1% by mass to 65% by mass, more preferably in a range from 1% by mass to 60% by mass, and even more preferably in a range from 1% by mass to 55% by mass.

When the content of SiO₂ in the ion sustained-release glass is 1% by mass or more, a glass with high transparency can be easily obtained, and when the content of SiO₂ in the ion sustained-release glass is 65% by mass or less, a composition with appropriate curability can be easily obtained.

The content of fluorine (F) in the ion sustained-release glass is not particularly limited. The content of fluorine in the ion sustained-release glass is in a range from 1% by mass to 30% by mass, preferably in a range from 3% by mass to 25% by mass, and more preferably in a range from 4% by mass to 20% by mass.

When the content of F in the ion sustained-release glass is 1% by mass or more, the sustained-release of fluoride ions in the dental composition can be enhanced, the effect of inhibiting dentin demineralization can be improved, and the effect of preventing caries can be imparted. Moreover, when the content of F in the ion sustained-release glass is 30% by mass or less, the viscosity of the ion sustained-release glass can be easily adjusted.

In addition, the ion sustained-release glass is preferably a glass that does not substantially contain phosphorus or a glass that contains a small amount of phosphorus. In this specification, "does not substantially contain" means that a subject component, such as phosphorus, is not intentionally blended.

The subject component of "does not substantially contain" may be included in the ion sustained-release glass as an inevitable impurity. In addition, the content of the subject component as the inevitable impurity is preferably less than 2% by mass. When a small amount of phosphorus is included in the ion sustained-release glass, the content of phosphoric acid (P₂O₅) in terms of oxide is preferably 5% by mass or less.

In the present embodiment, since the ion sustained-release glass does not substantially contain phosphorus, cations such as zinc ions and calcium ions released slowly are not trapped by phosphate ions; accordingly, the sustained-release property of cations such as zinc ions and calcium ions can be improved.

The ion sustained-release glass is more preferably a glass that does not substantially contain sodium. In the present embodiment, since the ion sustained-release glass does not substantially contain sodium, cations such as zinc ions and calcium ions that are released slowly are not trapped by sodium; accordingly, the sustained-release of cations such as zinc ions and calcium ions can be improved.

The contained amount of the ion sustained-release glass in the ion sustained-release composite particles is, for example, preferably in a range from 40% by mass to 90% by mass, more preferably in a range from 50% by mass to 80% by mass, and even more preferably in a range from 60% by mass to 70% by mass.

When the contained amount of ion sustained-release glass in the ion sustained-release composite particles is 40% by mass or more, the ion sustained-release effect from the composition containing the ion sustained-release composite particles is easily obtained. When the contained amount of ion sustained-release glass in the ion sustained-release composite particles is 90% by mass or less, the ion sustained-release composite particles are easily produced.

The form of the ion sustained-release glass is preferably powder (or particulate material). If the ion sustained-release glass is powder, the ion sustained-release glass is easily incorporated into a dental composition.

The particle size of the ion sustained-release glass in median diameter is in a range from 0.02 um to 30 um, more preferably in a range from 0.02 um to 25 um, and more preferably in a range from 0.02 um to 20 um. Here, the particle size means the average particle size defined by the median diameter.

When the particle size of the ion sustained-release glass is 0.02 um or more, the ion sustained-release glass powder for a dental composition improves the workability of the ion sustained-release composite particles when used as a glass powder for the dental composition. When the particle size of the ion sustained-release glass is 30 um or less, the wear resistance of a cured body of the dental composition is improved.

The polymer compound contained in the ion sustained-release composite particles contains a homo polymer or a copolymer of a (meth)acrylate compound having a hydroxyl group. In this specification, (meth)acrylate refers to at least one selected from acrylate and methacrylate.

In this specification, the homo polymer refers to a polymer mainly consisting of the constituent units of a certain polymeric component. The copolymer refers to a polymer obtained by copolymerizing the constituent units of a certain polymeric component with the constituent units of other polymeric components. It should be noted that the homo polymer and the copolymer may contain other polymeric components that are inevitably mixed.

By including a homo polymer or copolymer of a (meth)acrylate compound having a hydroxyl group in the polymer compound contained in the ion sustained-release composite particles, the ion sustained-release property can be favorably maintained while suppressing the acid reactivity of the ion sustained-release glass. Thus, when the ion sustained-release composite particles are used in the dental composition, shortening of the curing time of the dental composition can be suppressed while maintaining the favorable ion sustained-release property of the ion sustained-release glass.

Examples of (meth)acrylate compounds having a hydroxyl group include hydroxyethyl methacrylate (HEMA), glycerol dimethacrylate (GDMA), bisphenol A diglycidyl methacrylate (Bis-GMA), and the like. One or more of these (meth)acrylate compounds having hydroxyl groups may be used in combination.

The content of homo polymer or copolymer of (meth)acrylate compound having a hydroxyl group in the polymer compound contained in the ion sustained-release composite particles is not particularly limited. For example, the content of homo polymer or copolymer of (meth)acrylate compound having a hydroxyl group in the polymer compound contained in the ion sustained-release composite particles is in a range from 50% by mass to 100% by mass, preferably in a range from 60% by mass to 100% by mass, and more preferably in a range from 70% by mass to 100% by mass.

When the content of homo polymer or copolymer of (meth)acylate compound having a hydroxyl group in the polymer compound contained in the ion sustained-release composite particles is 50% by mass or more, the acid reactivity of the ion sustained-release glass can be sufficiently suppressed.

The copolymer of the (meth)acrylate compound having a hydroxyl group may contain monomer units of other (meth)acrylate compounds other than the (meth)acrylate compound having a hydroxyl group.

Other than the (meth)acrylate having a hydroxyl group, other (meth)acrylate compounds included as monomer units include, for example, multifunctional (meth)acrylate compounds, of which a bifunctional (meth)acrylate compound is preferably used.

Examples of the bifunctional (meth)acrylate compounds include ethoxylated bisphenol A dimethacrylate, di-2-methacryloyloxyethyl 2,2,4-trimethyl hexamethylene dicarbamate (UDMA), triethylene glycol dimethacrylate (TEGDMA), and the like. One or more of these bifunctional (meth)acrylate compounds may be used in combination.

The content of the bifunctional (meth)acrylate compound contained as monomer units in the copolymer of (meth)acrylate compound having a hydroxyl group in the polymer compound contained in the ion sustained-release composite particles is not particularly limited and can be, for example, in a range from 1% by mass to 40% by mass, preferably in a range from 5% by mass to 35% by mass, and more preferably in a range from 10% by mass to 30% by mass.

When the polymer compound contained in the ion sustained-release composite particles contains the monomer units of bifunctional (meth)acrylate as another (meth)acrylate compound other than the (meth)acrylate compound having a hydroxyl group, flexibility can be imparted to a product using the ion sustained-release composite particles while maintaining the mechanical strength of the ion sustained-release composite particles.

The contained amount of the homo polymer or copolymer of the (meth)acrylate compound having a hydroxyl group in the ion sustained-release composite particles is, for example, preferably in a range from 10% by mass to 45% by mass, more preferably in a range from 15% by mass to 40% by mass, and even more preferably in a range from 20% by mass to 30% by mass.

When the contained amount of homo polymer or copolymer of the (meth)acrylate compound having a hydroxyl group in the ion sustained-release composite particles is 10% by mass or more, the effect of suppressing the acid reactivity of the ion sustained-release composite particles is improved. When the contained amount of homo polymer or copolymer of the (meth)acrylate compound having a hydroxyl group in the ion sustained-release composite particles is 45% by mass or less, the ion sustained-release property from the ion sustained-release composite particles is improved.

The ion sustained-release composite particles of the present embodiment may contain other components as long as it does not impair the purpose of the present invention. Other components contained in the ion sustained-release composite particles include, for example, fillers, polymerization initiators, and polymerization inhibitors.

Components of the fillers are not particularly limited, but inorganic fillers are preferably used, such as colloidal silica, fine particle silica whose surface is hydrophobized, aluminum oxide (excluding aluminum oxide in the ion sustained-release glass), and the like. One or more of these fillers may be used, or two or more may be used in combination.

The contained amount of filler in the ion sustained-release composite particles is, for example, preferably in a range from 0.01% by mass to 30% by mass, more preferably in a range from 0.05% by mass to 20% by mass, and even more preferably in a range from 0.1% by mass to 10% by mass.

When the contained amount of filler in the ion sustained-release composite particles is 0.01% by mass or more, the dispersibility of the ion sustained-release glass in the dental composition is improved. When the contained amount of filler in the ion sustained-release composite particles is 30% by mass or less, the contained amount of ion sustained-release glass can be increased.

Examples of the polymerization initiators include, but are not particularly limited to, azobisisobutyronitrile (AIBN) and the like.

The content of the polymerization initiator in the ion sustained-release composite particles is not particularly limited. For example, the content of the polymerization initiator in the ion sustained-release composite particles is in a range from 0.01% by mass to 3% by mass, preferably in a range from 0.03% by mass to 2% by mass, more preferably in a range from 0.05% by mass to 1% by mass. When the content of the polymerization initiator in the ion sustained-release composite particles is in a range from 0.01% by mass to 3% by mass, the effect of suppressing the acid reactivity of the ion sustained-release glass is stabilized, and the ion sustained-release property of the ion sustained-release glass is stabilized.

The polymerization inhibitor is not particularly limited, but is for example, 2,6-di-tert-butyl-p-cresol and the like.

The content of the polymerization inhibitor in the ion sustained-release composite particles is not particularly limited, but is for example, in a range from 0.01% by mass to 5% by mass, preferably in a range from 0.05% by mass to 3% by mass, and more preferably in a range from 0.1% by mass to 2% by mass. When the content of the polymerization inhibitor in the ion sustained-release composite particles is in a range from 0.01% by mass to 5% by mass, the effect of suppressing the acid reactivity of the ion sustained-release glass is stabilized, and the ion sustained-release property of the ion sustained-release glass is stabilized.

The use of the ion sustained-release composite particles in the present embodiment is not particularly limited, but the ion sustained-release composite particles can be used for various dental materials, for example. Here, examples of the dental materials include dental cement, dental adhesive, dental temporary sealing material, dental primer, dental coating material, dental composite, dental hard resin, dental cutting resin material, dental temporary restorative material, dental filler, toothpaste, and the like. Among these, the ion sustained-release composite particles are suitably used for dental temporary sealing material.

### <Method of Producing Ion Sustained-Release Composite Particles>

A method of producing the ion sustained-release composite particles of the present embodiment is a method of producing the ion sustained-release composite particles containing the ion sustained-release glass and the polymer compound. The method includes a step of polymerizing and curing a mixture containing the ion sustained-release glass and a (meth)acrylate compound having a hydroxyl group. The method of producing the ion sustained-release composite particles of the present embodiment is substantially the method of producing the ion sustained-release composite particles of the present embodiment described above.

For the ion sustained-release glass used in the method of producing the ion sustained-release composite particles of the present embodiment and the polymer compound that is a (meth)acrylate compound having hydroxyl group, the ion sustained-release glass contained in the ion sustained-release composite particles of the present embodiment described above and the (meth)acrylate compound having hydroxyl group can be used.

In the method of producing ion sustained-release composite particles of the present embodiment, in the step of polymerizing and curing a mixture (hereinafter referred to as "polymerization step") containing ion sustained-release glass and a (meth)acrylate compound having a hydroxyl group (hereinafter referred to as "mixture"), the mixture further preferably contains a polymerization initiator.

Examples of the polymerization initiators include, but are not particularly limited to, azobisisobutyronitrile (AIBN) and the like.

The mixture further preferably contains a polymerization inhibitor.

Examples of the polymerization inhibitors include, but are not limited to, 2,6-di-tert-butyl-p-cresol and the like.

The method of producing the ion sustained-release composite particles of the present embodiment further includes a silane treatment step. The silane treatment step is a step of silane-treating the ion sustained-release glass contained in the ion sustained-release composite particles with a silane treatment agent.

Examples of the silane treatment agents include, but are not limited to, 3-methacryloyloxy propyltrimethoxysilane and the like.

In the method of producing the ion sustained-release composite particles, the mixture is polymerized and cured by the polymerization step to obtain a cured body (hereinafter referred to as "polymerized and cured body"). The polymerized and cured body contains the ion sustained-release glass and a homo polymer or copolymer of a (meth)acrylate compound having a hydroxyl group. The polymerized and cured body becomes ion sustained-release composite particles by pulverization.

The pulverization method is not particularly limited, and for example, a stamp mill, a planetary mill or the like can be used.

The polymerized and cured body that has been pulverized (pulverized product) can be passed through a sieve to obtain ion sustained-release composite particles adjusted to the desired particle size.

The particle size of the ion sustained-release glass to be adjusted is not particularly limited, but is, for example, in a range from 0.02 um to 30 um in median diameter, more preferably in a range from 0.02 um to 25 um in median diameter, and even more preferably 0.02 um to 20 um in median diameter.

### EXAMPLES

The present invention will be described below with further examples. In the following, the unitless value or "%" is the mass standard (% by mass) unless otherwise noted.

### <Ion Sustained-Release Glass>

Glass Examples 1 to 6 with the compositions indicated in Table 1 were prepared, and each glass was pulverized into a glass powder with a median diameter of 0.4 µm.

**[Table 1]**

| | Glass Example 1 | Glass Example 2 | Glass Example 3 | Glass Example 4 | Glass Example 5 | Glass Example 6 |
|---|---|---|---|---|---|---|
| Al₂O₃ | 0.5 | 14.3 | 12.8 | 17.4 | 19.8 | 19.2 |
| ZnO | 24.0 | 32.1 | 25.0 | 15.1 | 0.0 | 0.0 |
| SrO | 0.0 | 0.0 | 0.0 | 0.0 | 27.0 | 35.6 |
| CaO | 6.8 | 9.0 | 9.0 | 12.3 | 0.0 | 0.1 |
| La₂O₃ | 35.1 | 0.0 | 0.0 | 0.0 | 6.3 | 0.0 |
| SiO₂ | 29.0 | 39.0 | 47.1 | 50.4 | 29.8 | 25.7 |
| P₂O₅ | 0.0 | 0.0 | 0.0 | 0.0 | 3.6 | 4.1 |
| F | 4.3 | 5.6 | 6.1 | 4.8 | 11.0 | 14.1 |
| Na₂O | 0.0 | 0.0 | 0.0 | 0.0 | 2.5 | 1.2 |

### <Silane-Treated Glass Powder>

The glass powder was annealed at 400°C for 2 hours, silane-treated with 4% of 3-methacryloyloxy propyltrimethoxysilane, and dried at 110°C for 3 hours to prepare silane-treated glass powder (Glass Examples 1 to 6).

### [Examples 1 to 10 and Comparative Examples 1 to 12]

Silane-treated glass powder (Glass Examples 1 to 6) and other components (polymer solution and R812) were mixed with the compositions indicated in Tables 2 to 13, defoamed at 2000 rpm and 10 kPa, poured into a silicone mold, and heated at 90°C for 3 hours to polymerize. The component indicated as R812 in Tables 2, 4, 6, 8, 10 and 12 is fine silica that is surface-treated with hexamethyldisilazane (Aerosil (Registered Trademark) R812, manufactured by Aerosil Japan Ltd.). The resulting polymer (cured polymer body) was pulverized by a stamp mill for 2 minutes and by a planetary mill at 150 rpm for 60 minutes and passed through a 200-mesh sieve to obtain composite powders (composite particles) of Examples 1 to 10 and Comparative Examples 1 to 12. Comparative Examples 2, 4, 6, 8, 10, and 12 were obtained by using Glass Examples 1 to 6 as they were, without performing compositing with a polymer compound.

**[Table 2]**

| | Example 1 | Example 2 | Example 3 | Example 4 | Example 5 | Comparative Example 1 | Comparative Example 2 |
|---|---|---|---|---|---|---|---|
| Glass Example 1 | 73% | 73% | 73% | 73% | 73% | 74% | 100% |
| Glass Example 2 | | | | | | | |
| Glass Example 3 | | | | | | | |
| Glass Example 4 | | | | | | | |
| Glass Example 5 | | | | | | | |
| Glass Example 6 | | | | | | | |
| Polymer Solution | 24% | 24% | 24% | 24% | 24% | 25% | - |
| R812 | 3% | 3% | 3% | 3% | 3% | 1% | - |

**[Table 3]**

| | Example 1 | Example 2 | Example 3 | Example 4 | Example 5 | Comparative Example 1 | Comparative Example 2 |
|---|---|---|---|---|---|---|---|
| 2-hydroxyethyl methacrylate | 49.58% | - | 79.33% | 59.49% | 99.16% | - | - |
| 2-hydroxy-1,3-dimethacryloxypropane (GDMA) | 29.75% | 79.33% | - | 39.66% | - | - | - |
| Ethoxylated bisphenol A dimethacrylate (m+n ≒ 30) | 19.83% | 19.83% | 19.83% | - | - | - | - |
| Ethoxylated bisphenol A dimethacrylate (m+n ≒ 2.6) | - | - | - | - | - | 49.58% | |
| di-2-methacryloyloxyethyl 2,2,4-trimethylhexamethylenedicarbamate (UDMA) | - | - | - | - | - | 9.92% | - |
| Triethylene glycol dimethacrylate (TEGDMA) | - | - | - | - | - | 39.66% | - |
| 2,6-di-tert-butyl-p-cresol | 0.10% | 0.10% | 0.10% | 0.10% | 0.10% | 0.10% | - |
| Azobisisobutyronitrile | 0.74% | 0.74% | 0.74% | 0.74% | 0.74% | 0.74% | - |

**[Table 4]**

| | Example 6 | Comparative Example 3 | Comparative Example 4 |
|---|---|---|---|
| Glass Example 1 | | | |
| Glass Example 2 | 73% | 74% | 100% |
| Glass Example 3 | | | |
| Glass Example 4 | | | |
| Glass Example 5 | | | |
| Glass Example 6 | | | |
| Polymer solution | 24% | 25% | - |
| R812 | 3% | 1% | - |

**[Table 5]**

| | Example 6 | Comparative Example 3 | Comparative Example 4 |
|---|---|---|---|
| 2-hydroxyethyl methacrylate | 49.58% | | - |
| 2-hydroxy-1,3-dimethacryloxypropane (GDMA) | 29.75% | | - |
| Ethoxylated bisphenol A dimethacrylate (m+n ≒ 30) | 19.83% | | - |
| Ethoxylated bisphenol A dimethacrylate (m+n ≒ 2.6) | | 49.58% | |
| di-2-methacryloyloxyethyl 2,2,4-trimethylhexamethylenedicarbamate (UDMA) | | 9.92% | - |
| Triethylene glycol dimethacrylate (TEGDMA) | | 39.66% | - |
| 2,6-di-tert-butyl-p-cresol | 0.10% | 0.10% | - |
| Azobisisobutyronitrile | 0.74% | 0.74% | - |

**[Table 6]**

| | Example 7 | Comparative Example 5 | Comparative Example 6 |
|---|---|---|---|
| Glass Example 1 | | | |
| Glass Example 2 | | | |
| Glass Example 3 | 73% | 74% | 100% |
| Glass Example 4 | | | |
| Glass Example 5 | | | |
| Glass Example 6 | | | |
| Polymer solution | 24% | 25% | - |
| R812 | 3% | 1% | - |

**[Table 7]**

| | Example 7 | Comparative Example 5 | Comparative Example 6 |
|---|---|---|---|
| 2-hydroxyethyl methacrylate | 49.58% | | - |
| 2-hydroxy-1,3-dimethacryloxypropane (GDMA) | 29.75% | | - |
| Ethoxylated bisphenol A dimethacrylate (m+n ≒ 30) | 19.83% | | - |
| Ethoxylated bisphenol A dimethacrylate (m + n ≒ 2.6) | | 49.58% | |
| di-2-methacryloyloxyethyl 2,2,4-trimethylhexamethylenedicarbamate (UDMA) | | 9.92% | - |
| Triethylene glycol dimethacrylate (TEGDMA) | | 39.66% | - |
| 2,6-di-tert-butyl-p-cresol | 0.10% | 0.10% | - |
| Azobisisobutyronitrile | 0.74% | 0.74% | - |

**[Table 8]**

| | Example 8 | Comparative Example 7 | Comparative Example 8 |
|---|---|---|---|
| Glass Example 1 | | | |
| Glass Example 2 | | | |
| Glass Example 3 | | | |
| Glass Example 4 | 73% | 74% | 100% |
| Glass Example 5 | | | |
| Glass Example 6 | | | |
| Polymer solution | 24% | 25% | - |
| R812 | 3% | 1% | - |

**[Table 9]**

| | Example 8 | Comparative Example 7 | Comparative Example 8 |
|---|---|---|---|
| 2-hydroxyethyl methacrylate | 49.58% | | - |
| 2-hydroxy-1,3-dimethacryloxypropane (GDMA) | 29.75% | | - |
| Ethoxylated bisphenol A dimethacrylate (m+n ≒ 30) | 19.83% | | - |
| Ethoxylated bisphenol A dimethacrylate (m+n ≒ 2.6) | | 49.58% | |
| di-2-methacryloyloxyethyl 2,2,4-trimethylhexamethylenedicarbamate (UDMA) | | 9.92% | - |
| Triethylene glycol dimethacrylate (TEGDMA) | | 39.66% | - |
| 2,6-di-tert-butyl-p-cresol | 0.10% | 0.10% | - |
| Azobisisobutyronitrile | 0.74% | 0.74% | - |

**[Table 10]**

| | Example 9 | Comparative Example 9 | Comparative Example 10 |
|---|---|---|---|
| Glass Example 1 | | | |
| Glass Example 2 | | | |
| Glass Example 3 | | | |
| Glass Example 4 | | | |
| Glass Example 5 | 73% | 74% | 100% |
| Glass Example 6 | | | |
| Polymer solution | 24% | 25% | - |
| R812 | 3% | 1% | - |

**[Table 11]**

| | Example 9 | Comparative Example 9 | Comparative Example 10 |
|---|---|---|---|
| 2-hydroxyethyl methacrylate | 49.58% | | - |
| 2-hydroxy-1,3-dimethacryloxypropane (GDMA) | 29.75% | | - |
| Ethoxylated bisphenol A dimethacrylate (m+n ≒ 30) | 19.83% | | - |
| Ethoxylated bisphenol A dimethacrylate (m + n ≒ 2.6) | | 49.58% | |
| di-2-methacryloyloxyethyl 2,2,4-trimethylhexamethylenedicarbamate (UDMA) | | 9.92% | - |
| Triethylene glycol dimethacrylate (TEGDMA) | | 39.66% | - |
| 2,6-di-tert-butyl-p-cresol | 0.10% | 0.10% | - |
| Azobisisobutyronitrile | 0.74% | 0.74% | - |

**[Table 12]**

| | Example 10 | Comparative Example 11 | Comparative Example 12 |
|---|---|---|---|
| Glass Example 1 | | | |
| Glass Example 2 | | | |
| Glass Example 3 | | | |
| Glass Example 4 | | | |
| Glass Example 5 | | | |
| Glass Example 6 | 73% | 74% | 100% |
| Polymer solution | 24% | 25% | - |
| R812 | 3% | 1% | - |

**[Table 13]**

| | Example 10 | Comparative Example 11 | Comparative Example 12 |
|---|---|---|---|
| 2-hydroxyethyl methacrylate | 49.58% | | - |
| 2-hydroxy-1,3-dimethacryloxypropane (GDMA) | 29.75% | | - |
| Ethoxylated bisphenol A dimethacrylate (m+n ≒ 30) | 19.83% | | - |
| Ethoxylated bisphenol A dimethacrylate (m + n ≒ 2.6) | | 49.58% | |
| di-2-methacryloyloxyethyl 2,2,4-trimethylhexamethylenedicarbamate (UDMA) | | 9.92% | - |
| Triethylene glycol dimethacrylate (TEGDMA) | | 39.66% | - |
| 2,6-di-tert-butyl-p-cresol | 0.10% | 0.10% | - |
| Azobisisobutyronitrile | 0.74% | 0.74% | - |

The resulting composite powder (Examples 1 to 10, Comparative Examples 1 to 12) was evaluated for ion sustained-release and acid reactivity. The various tests and evaluations that were conducted are according to the following methods.

### <Ion Elution Test of Composite Powder>

0.01 g of a composite powder was placed in a glass vial, 10 mL of a solution (pH 4.5) that was prepared by mixing 0.2 mol/L of a lactic-sodium lactate buffer and methanol in a ratio of 1:1 was added to the composite powder in the glass vial, stirred for 24 hours, and then centrifuged at 3000 rpm for 10 minutes. The amount of ions in the filtrate filtered through a 0.20 um filter was measured using an inductively coupled plasma (ICP) emission spectrometer (manufactured by Thermo Fisher). The types of ions measured were Zn²⁺, Ca²⁺, La³⁺, and Sr²⁺.

### <Ion Sustained-Release Property>

In the ion elution test of the composite powder, the detected amount of at least one types of ions of zinc ion (Zn²⁺), calcium ion (Ca²⁺), lanthanum ion (La³⁺), and strontium ion (Sr²⁺) was measured. Regarding the measured ion detected amount, for Examples 1 to 5 and Comparative Example 2, the percentage of increase was calculated based on the ion detected amount of Comparative Example 1 (Tables 2 and 3). For Examples 6 to 10 and Comparative Examples 4, 6, 8, 10, and 12, the percentage increase was calculated based on Comparative Examples 3, 5, 7, 9, and 11, respectively (Tables 4 to 13). Ion sustained-release property was evaluated by using the following evaluation criteria. It should be noted that Comparative Examples 1, 3, 5, 7, 9, and 11 were evaluated as not acceptable. The results of ion sustained-release property are indicated in Tables 14 to 19. Good: When the percentage increase was 10% or higher Not good: When the percentage increase was less than 10%

### <Acid Reactivity>

A composite powder and an ion-releasing filling material (CAREDYNE (Registered Trademark) RESTORE liquid, manufactured by GC Corporation) were mixed in a 1:1 powder-liquid ratio in a thermostatic chamber at a temperature of 23°C and a humidity of 50%, and then kneaded for 30 seconds to obtain a kneaded product. The kneaded product was filled in a ring with 8 mm in diameter and 2 mm in height, and the exothermic peak of the kneaded product was observed by an infrared radiation pyrometer. Acid reactivity was evaluated by using the following evaluation criteria. The results of acid reactivity are indicated in Tables 14 to 19. Good: Peak temperature is less than 50% of Comparative Examples 2, 4, 6, 8, 10, or 12 (in the same Glass Example without performing compositing with the polymer compound)
Not good: Peak temperature is 50% or higher of Comparative Examples 2, 4, 6, 8, 10, or 12 (in the same Glass Example without performing compositing with the polymer compound)

**[Table 14]**

| | Example 1 | Example 2 | Example 3 | Example 4 | Example 5 | Comparative Example 1 | Comparative Example 2 |
|---|---|---|---|---|---|---|---|
| Ion sustained-release property | Good | Good | Good | Good | Good | Not good | Good |
| Acid reactivity | Good | Good | Good | Good | Good | Good | Not good |

**[Table 15]**

| | Example 6 | Comparative Example 3 | Comparative Example 4 |
|---|---|---|---|
| Ion sustained-release property | Good | Not good | Good |
| Acid reactivity | Good | Good | Not good |

**[Table 16]**

| | Example 7 | Comparative Example 5 | Comparative Example 6 |
|---|---|---|---|
| Ion sustained-release property | Good | Not good | Good |
| Acid reactivity | Good | Good | Not good |

**[Table 17]**

| | Example 8 | Comparative Example 7 | Comparative Example 8 |
|---|---|---|---|
| Ion sustained-release property | Good | Not good | Good |
| Acid reactivity | Good | Good | Not good |

**[Table 18]**

| | Example 9 | Comparative Example 9 | Comparative Example 10 |
|---|---|---|---|
| Ion sustained-release property | Good | Not good | Good |
| Acid reactivity | Good | Good | Not good |

**[Table 19]**

| | Example 10 | Comparative Example 11 | Comparative Example 12 |
|---|---|---|---|
| Ion sustained-release property | Good | Not good | Good |
| Acid reactivity | Good | Good | Not good |

Tables 14 to 19 indicate that the composite particles containing the ion sustained-release glass and the polymer compound, where the polymer compound is a (meth)acrylate compound having a hydroxyl group, all indicated good ion sustained-release properties and acid reactivities (Examples 1 to 10).

In contrast, the composite particles containing ion sustained-release glass and the polymer compound that does not contain a (meth)acrylate compound having a hydroxyl group were not capable of ion sustained-release properties (Comparative Examples 1, 3, 5, 7, 9, and 11).

In addition, the ion sustained-release glass that was not composited with polymer compound was acid-reactive (Comparative Examples 2, 4, 6, 8, 10, and 12) .

From these results, it was found that the ion sustained-release composite particles containing the ion sustained-release glass and a homo polymer or a copolymer of a (meth)acrylate compound having a hydroxyl group as the polymer compound suppressed acid reactivity and maintained ion sustained-release property.

As described above, the invention is not limited to a specific embodiment, and various modifications and changes are possible within the scope of the invention described in the claims.

The present application is based on and claims priority of Patent Application No. 2021-58605 filed March 30, 2021 with the Japan Patent Office, and the entire contents of Japanese Patent Application No. 2021-58605 are hereby incorporated by reference.

## Claims

1. Ion sustained-release composite particles comprising:
ion sustained-release glass; and
a polymer compound,
wherein the polymer compound includes a homo polymer or a copolymer of a (meth)acrylate compound having a hydroxyl group.

2. The ion sustained-release composite particles according to claim 1, wherein the ion sustained-release glass contains at least one of zinc, calcium, lanthanum, and strontium.

3. The ion sustained-release composite particles according to claim 1 or 2, wherein the copolymer of the (meth)acrylate compound having a hydroxyl group contains a monomer unit of a bifunctional (meth)acrylate compound.

4. A method of producing ion sustained-release composite particles containing ion sustained-release glass and a polymer, the method comprising,
a step of polymerizing and curing a mixture of the ion sustained-release glass and a (meth)acrylate compound having a hydroxyl group.
